(19) Europäisches Patentamt
European Patent Office
Office européen des brevets

(11) Veröffentlichungsnummer: **0 289 919 B1**

(12) **EUROPÄISCHE PATENTSCHRIFT**

(45) Veröffentlichungstag der Patentschrift: **17.03.93**

(21) Anmeldenummer: **88106696.3**

(22) Anmeldetag: **27.04.88**

(51) Int. Cl.5: **C07D 233/68**, C07D 233/56, C07D 231/12, C07D 231/16, C07D 249/08, C07D 249/10, C07D 231/56, C07D 235/06, C07D 235/08, A01N 43/50, A01N 43/52

(54) **N-substituierte Azole.**

(30) Priorität: **02.05.87 DE 3714709**
**23.01.88 DE 3801919**

(43) Veröffentlichungstag der Anmeldung:
**09.11.88 Patentblatt 88/45**

(45) Bekanntmachung des Hinweises auf die Patenterteilung:
**17.03.93 Patentblatt 93/11**

(84) Benannte Vertragsstaaten:
**AT BE CH DE ES FR GB GR IT LI NL**

(56) Entgegenhaltungen:
**EP-A- 0 132 606**
**EP-A- 0 175 652**
**EP-B- 0 113 570**

**CHEMICAL ABSTRACTS, Band 106, Nr. 3, 19. Januar 1987, Columbus, Ohio, US; SUMITOMO CHEMICAL CO. "Preparation of benzimidazole derivatives as pesticides" Seite 607, Spalte 2, Zusammenfassung-Nr. 18 545y**

(73) Patentinhaber: **BASF Aktiengesellschaft**
**Carl-Bosch-Strasse 38**
**W-6700 Ludwigshafen(DE)**

(72) Erfinder: **Buerstinghaus, Rainer, Dr.**
**Robert-Koch-Strasse 2**
**W-4404 Telgte(DE)**
Erfinder: **Neubauer, Hans-Juergen, Dr.**
**B 4,2**
**W-6800 Mannheim(DE)**
Erfinder: **Hofmeister, Peter, Dr.**
**Bernard-Humblot-Strasse 12**
**W-6730 Neustadt(DE)**
Erfinder: **Kuenast, Christoph, Dr.**
**Salierstrasse 2**
**W-6701 Otterstadt(DE)**
Erfinder: **Leyendecker, Joachim, Dr.**
**Scharnhorststrasse 12**
**W-6800 Mannheim(DE)**
Erfinder: **Kardorff, Uwe, Dr.**
**Frankenthaler Strasse 42**
**W-6710 Frankenthal 6(DE)**

**EP 0 289 919 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 104, Nr. 21, 26. Mai 1986, Columbus, Ohio, US; SUMITOMO CHEMICAL CO. "Benzimidazole derivatives" Seite 634, Spalte 1, Zusammenfassung-Nr. 186 419n

## Beschreibung

Die vorliegende Erfindung betrifft neue N-substituierte Azole der allgemeinen Formel I

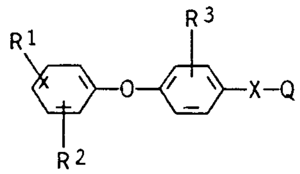

(I) ,

in der die Substituenten folgende Bedeutung haben:

| | |
|---|---|
| $R^1$, $R^2$, $R^3$ | Wasserstoff, Halogen, $C_1$-$C_8$-Alkyl, $C_1$-$C_8$-Alkoxy, $C_1$-$C_4$-Halogenalkyl, $C_1$-$C_4$-Halogenalkoxy, $C_3$-$C_{10}$-Cycloalkyl, Nitro oder Cyano, |
| Q | ein gegebenenfalls substituierter Azolrest der Formeln IIa-IId |

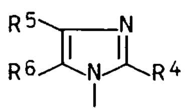

(IIa)

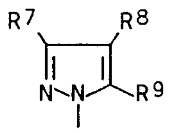

(IIb)

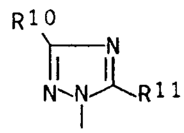

(IIc)

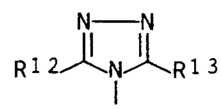

(IId)

in denen

| | |
|---|---|
| $R^4$ bis $R^{13}$ | Wasserstoff, Halogen, $C_1$-$C_8$-Alkyl, $C_1$-$C_4$-Halogenalkyl, $C_1$-$C_4$-Alkoxy, $C_3$-$C_{10}$-Cycloalkyl, Phenyl, 1-Naphthyl oder 2-Naphthyl, wobei diese aromatischen Reste gegebenenfalls ein- bis dreifach durch Halogen, $C_1$-$C_8$-Alkyl, $C_1$-$C_8$-Alkoxy, $C_1$-$C_4$-Halogenalkyl oder $C_1$-$C_4$-Halogenalkoxy substituiert sind, bedeuten, |
| X | im Falle des Azolrestes IIa $-CH_2-$ oder $-O(CH_2)_n-$, wobei n für 1, 2 oder 3 steht, im Falle der Azolreste IIb-IId $-OCH_2-$. |

Außerdem betrifft die Erfindung die Herstellung der Verbindungen I, Schädlingsbekämpfungsmittel, die die Verbindungen I als Wirkstoffe enthalten sowie ein Verfahren zur Bekämpfung von Schädlingen.

Aus der EP-A-132 606 und den Kurzreferaten C.A. 104, 634 (1986), 186 418m, und C.A. 106, 607 (1987), 18 545y sind N-substituierte Azole als insektizide und akarizide Wirkstoffe bekannt. Die Wirkung dieser Verbindungen läßt jedoch zu wünschen übrig. Des weiteren werden in der EP-A 175 652 ähnliche Pyrimidinderivate und in der EP-A 113 570 ähnliche Imidazolderivate beschrieben.

Der Erfindung lag daher die Aufgabe zugrunde, neue N-substituierte Azole I mit verbesserter Wirkung bereitzustellen.

Demgemäß wurden die eingangs definierten neuen N-substituierten Azole I sowie Verfahren zu deren Herstellung gefunden. Ferner wurde gefunden, daß sich die Verbindungen I hervorragend zur Bekämpfung von Schädlingen eignen.

Die Verbindungen I sind nach folgenden Methoden erhältlich:

a) Zur Herstellung der Verbindungen I, bei denen X $-CH_2-$ bedeutet, setzt man eine p-Phenoxybenzylverbindung III mit einem Anion eines Imidazoles IV nach folgender Reaktionsgleichung um:

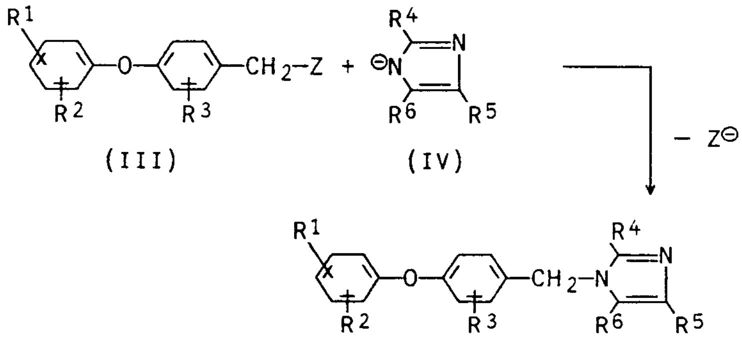

(III) (IV)

(Ia)

Die p-Phenoxybenzylverbindungen III sind zum Teil literaturbekannt (z.B. aus GB-A-1 140 748, DE-A-24 18 572, EP-A-132 606) oder können in an sich bekannter Weise hergestellt werden.

Die Anionen der Imidazole IV lassen sich aus literaturbekannten bzw. handelsüblichen Imidazolen IVa nach üblichen Methoden in Form entsprechender Salze, wie dem Natrium- oder Kaliumsalz, generieren.

Die Verbindungen Ia können auch durch Umsetzung von III mit einem Imidazol IVa

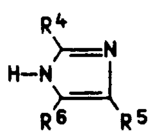

(IVa)

in Gegenwart einer Base erhalten werden. Man setzt normalerweise mindestens equivalente Mengen einer Base zu III und/oder IVa zu, kann aber diese auch im Überschuß oder gegebenenfalls auch als Lösungsmittel verwenden. Als Basen eignen sich beispielsweise Alkoholate von Alkalimetallen, wie Natriummethylat, Natriumethylat, oder Kalium-tert.-butylat; Alkali- oder Erdalkalimetallhydride, wie Natriumhydrid, Kaliumhydrid oder Calciumhydrid; Alkalicarbonate, wie Natriumcarbonat, Kaliumcarbonat; aliphatische Amine, wie Dimethylamin, Triethylamin oder Diisopropylamin; heterocyclische Amine, wie Piperidin, Piperazin oder Pyrrolidin; aromatische Amine, wie Pyridin oder Pyrrol, und gegebenenfalls auch Alkyllithium-Verbindungen, wie n-Butyllithium.

b) Zur Herstellung der Verbindungen I, bei denen X $-O(CH_2)_2-$ oder $-O(CH_2)_3-$ bedeutet, setzt man eine p-Phenoxyphenoxyverbindung V mit einem Anion eines Imidazoles IV nach folgender Reaktionsgleichung um:

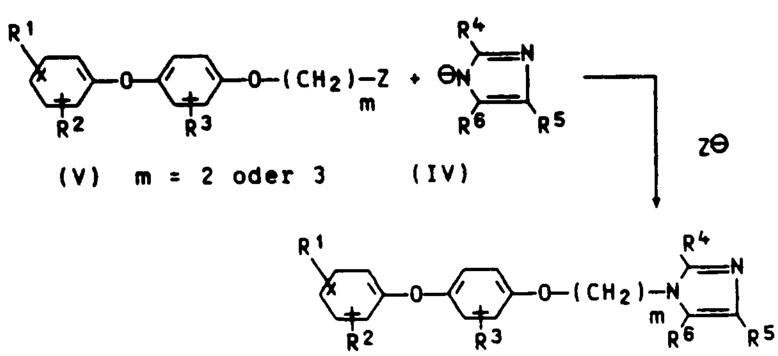

(V) m = 2 oder 3 (IV)

(Ia)

c) Zur Herstellung der Verbindungen I, bei denen X $-OCH_2-$ bedeutet, setzt man p-Phenoxyphenole VI mit einem N-Methylazol VII in Gegenwart einer Base im Temperaturbereich von (-20) bis 250 °C, vorzugsweise von 20 bis 120 °C gemäß folgender Reaktionsgleichung um:

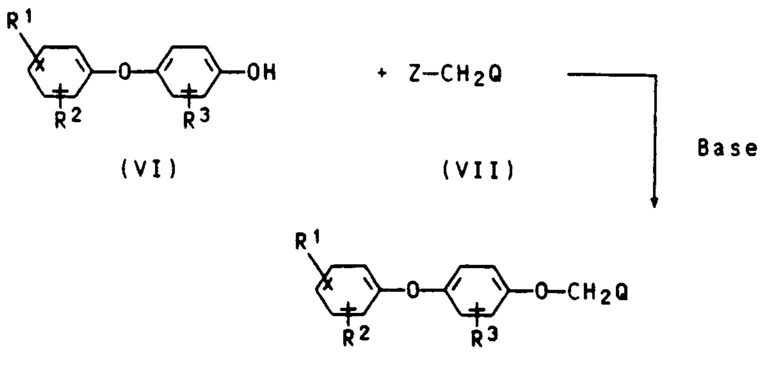


(VI) (VII) (I)

Die p-Phenoxyphenole VI sind zum Teil aus Houben-Weyl, Bd. VI, 3, Methoden der org. Chemie, Thieme Verlag, 1965, 585 ff bekannt oder können nach den dort beschriebenen Methoden hergestellt werden.

Die N-Methylazole VII sind zum Teil aus Heterocycles 24, 2233 (1986) bekannt, oder können nach der dort beschriebenen Methode gemäß folgender Reaktionsgleichung hergestellt werden:

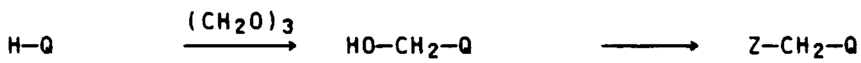


(VIIa) (VII)

c2) Zur Herstellung der Verbindungen I, in denen X = $-OCH_2$ bedeutet, setzt man ein Phenolatanion von VI mit einem N-Methylazol VII in einem Temperaturbereich von (-20) bis 120 ° C, vorzugsweise von (-20) bis 80 ° C und folgender Reaktionsgleichung um:

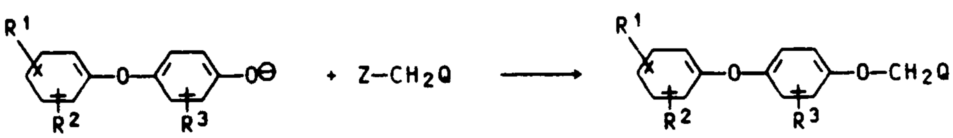

(VI) (VII) (I)

Die p-Phenoxyphenolatanionen VI sind in Form ihrer Metallsalze, wie dem Natrium oder Kaliumsalz, bekannt oder lassen sich aus den p-Phenoxyphenolen durch Umsetzung mit gängigen Metallierungsreagentien, wie Natriummethylat, Natriumethylat, Kalium-tert.-butylat, Natrium- bzw. Kaliumhydrid oder Butyllithium, generieren.

In allen vier Fallgestaltungen bedeutet der Rest Z eine Abgangsgruppe, wie beispielsweise einen Sulfonsäurerest oder ein Halogen. Unter den Sulfonsäureresten sind Methansulfonyl, Trifluormethansulfonyl und p-Toluolsulfonyl, unter den Halogenen sind Chlor und Brom bevorzugt; besonders bevorzugt wird Chlor.

Die Umsetzungen mit den jeweiligen Anionen der Metallsalze werden in den Fällen a), b), c1) und c2) zweckmäßigerweise in einem Lösungsmittel oder Verdünnungsmittel durchgeführt. Hierzu eignen sich beispielsweise aliphatische Kohlenwasserstoffe wie n-Pentan, n-Hexan, das Hexan-Isomerengemisch, Petrolether, Cyclohexan, Heptan, aromatische Kohlenwasserstoffe wie Benzol, Toluol, die Xylole und deren Isomerengemische, Benzin, chlorierte Kohlenwasserstoffe wie Methylenchlorid, Chloroform, Tetrachlorkohlenstoff, 1,2-Dichlormethan, chlorierte Aromaten wie Chlorbenzol, 1-Chlornaphthalin; Ether wie Diethyl- und Di-n-Butylether, Methyl-tert.-butylether, Tetrahydrofuran, Dioxan; Ketone wie Aceton, Methylethylketon und Methylisopropylketon; Nitrile wie Acetonitril und Propionitril; aprotische dipolare Lösungsmittel wie Dimethylformamid, Dimethylsulfoxid oder Pyridin. Auch Gemische dieser Stoffe können als Lösungs- oder Verdünnungsmittel verwendet werden.

Zur Herstellung der erfindungsgemäßen Verbindungen I nach den vorstehend beschriebenen Methoden setzt man die Ausgangsstoffe üblicherweise in stöchiometrischem Verhältnis ein. Ein Überschuß des einen oder anderen kann in Einzelfällen aber durchaus vorteilhaft sein.

Die Umsetzungen verlaufen gewöhnlich oberhalb von -30 °C mit ausreichenden Geschwindigkeiten. 100 °C müssen im allgemeinen nicht überschritten werden. Da sie in einigen Fällen unter Wärmeentwicklung verlaufen, kann es von Vorteil sein, Kühlmöglichkeiten vorzusehen.

Die Reaktionsgemische werden in üblicher Weise aufgearbeitet, z.B. durch Versetzen mit Wasser, Trennen der Phasen und Säulenchromatographie. Die neuen Verbindungen der Formel I fallen teilweise in Form farbloser oder schwach bräunlich gefärbter, zäher Öle an, die sich durch längeres Erwärmen unter vermindertem Druck auf mäßig erhöhte Temperatur ("Andestillieren") von den letzten flüchtigen Anteilen befreien und auf diese Weise reinigen lassen. Erhält man die Verbindungen der Formel I in kristalliner Form, so kann ihre Reinigung durch Umkristallisieren erfolgen.

Die Substituenten in Formel I haben im einzelnen folgende Bedeutungen:

$R^1$

- Wasserstoff,
- Halogen, bevorzugt Fluor, Chlor und Brom, besonders bevorzugt Fluor und Chlor in meta-Stellung,
- unverzweigtes oder verzweigtes $C_1$-$C_8$-Alkyl, bevorzugt unverzweigtes oder verzweigtes $C_1$-$C_6$-Alkyl, besonders bevorzugt unverzweigtes oder verzweigtes $C_1$-$C_4$-Alkyl in meta-Stellung wie m-Methyl, m-Ethyl, m-(n-Propyl), m-(iso-Propyl), m-(n-Butyl), m-(iso-Butyl), m-(sec-Butyl) und m-(tert.-Butyl),
- unverzweigtes oder verzweigtes $C_1$-$C_8$-Alkoxy, bevorzugt unverzweigtes oder verzweigtes $C_1$-$C_4$-Alkoxy, besonders bevorzugt $C_1$-$C_2$-Alkoxy in meta-Stellung wie m-Methoxy und m-Ethoxy,
- unverzweigtes oder verzweigtes $C_1$-$C_4$-Halogenalkyl, bevorzugt $C_1$-$C_2$-Fluor- und Chloralkyl, besonders bevorzugt m-Trifluormethyl und m-Trichlormethyl,
- unverzweigtes oder verzweigtes $C_1$-$C_4$-Halogenalkoxy, bevorzugt $C_1$-$C_2$-Fluor- und Chloralkoxy, besonders bevorzugt m-Trifluormethoxy und m-Trichlormethoxy,
- $C_3$-$C_{10}$-Cycloalkyl, bevorzugt $C_3$-$C_6$-Cycloalkyl, besonders bevorzugt m-Cyclopropyl, m-Cyclobutyl, m-Cyclopentyl und m-Cyclohexyl,
- Nitro,
- Cyano,

$R^2$, $R^3$ unabhängig voneinander

- Wasserstoff,
- Halogen, bevorzugt Fluor und Chlor,
- unverzweigtes oder verzweigtes $C_1$-$C_8$-Alkyl, bevorzugt -unverzweigtes oder verzweigtes $C_1$-$C_4$-Alkyl, besonders bevorzugt $C_1$-$C_2$-Alkyl, wie Methyl und Ethyl,
- unverzweigtes oder verzweigtes $C_1$-$C_8$-Alkoxy, bevorzugt unverzweigtes oder verzweigtes $C_1$-$C_4$-Alkoxy, besonders bevorzugt $C_1$-$C_2$-Alkoxy, wie Methoxy und Ethoxy,
- unverzweigtes oder verzweigtes $C_1$-$C_4$-Halogenalkyl, bevorzugt $C_1$-$C_2$-Fluor- und Chloralkyl, besonders bevorzugt Trifluormethyl und Trichlormethyl,
- unverzweigtes oder verzweigtes $C_1$-$C_4$-Halogenalkoxy, bevorzugt $C_1$-$C_2$-Fluor- und Chloralkoxy, besonders bevorzugt Trifluormethoxy und Trichlormethoxy,
- $C_3$-$C_{10}$-Cycloalkyl, bevorzugt $C_3$-$C_6$-Cycloalkyl, wie Cyclopropyl, Cyclobutyl, Cyclopentyl und Cyclohexyl, besonders bevorzugt Cyclopropyl,
- Nitro,
- Cyano,

$R^4$-$R^{13}$ unabhängig voneinander

- Wasserstoff,
- Halogen, bevorzugt Fluor und Chlor,
- unverzweigtes oder verzweigtes $C_1$-$C_8$-Alkyl, bevorzugt unverzweigtes oder verzweigtes $C_1$-$C_4$-Alkyl, besonders bevorzugt, $C_1$-$C_2$-Alkyl wie Methyl und Ethyl,
- unverzweigtes oder verzweigtes $C_1$-$C_4$-Halogenalkyl, bevorzugt $C_1$-$C_2$-Fluor- und Chloralkyl, besonders bevorzugt Trifluormethyl und Trichlormethyl,
- unverzweigtes oder verzweigtes $C_1$-$C_8$-Alkoxy, bevorzugt unverzweigtes oder verzweigtes $C_1$-$C_4$-Alkoxy, besonders bevorzugt $C_1$-$C_2$-Alkoxy wie Methoxy und Ethoxy,
- $C_3$-$C_{10}$-Cycloalkyl, bevorzugt $C_3$-$C_6$-Cycloalkyl wie Cyclopropyl, Cyclobutyl, Cyclopentyl und Cyclohexyl, besonders bevorzugt Cyclopropyl,
- Aryl, bevorzugt Phenyl, 1-Naphthyl und 2-Naphthyl, besonders bevorzugt Phenyl,
- ein- bis dreifach durch Halogen substituiertes Aryl, bevorzugt durch Fluor oder Chlor monosubstituiertes Phenyl wie 2-Fluorphenyl, 3-Fluorphenyl, 4-Fluorphenyl, 2-Chlorphenyl, 3-Chlorphenyl und 4-Chlorphenyl,

- ein- bis dreifach durch unverzweigtes oder verzweigtes $C_1$-$C_8$-Alkyl substituiertes Aryl, bevorzugt durch unverzweigtes oder verzweigtes $C_1$-$C_4$-Alkyl monosubstituiertes Phenyl, besonders bevorzugt durch $C_1$-$C_2$-Alkyl monosubstituiertes Phenyl wie 4-Methylphenyl und 4-Ethylphenyl,
- ein- bis dreifach durch unverzweigtes oder verzweigtes $C_1$-$C_8$-Alkoxy substituiertes Aryl, bevorzugt durch unverzweigtes oder verzweigtes $C_1$-$C_4$-Alkoxy monosubstituiertes Phenyl, besonders bevorzugt durch $C_1$-$C_2$-Alkoxy monosubstituiertes Phenyl wie 4-Methoxyphenyl und 4-Ethoxyphenyl,
- ein- bis dreifach durch unverzweigtes oder verzweigtes $C_1$-$C_4$-Halogenalkyl substituiertes Aryl, bevorzugt durch $C_1$-$C_2$-Fluor- und Chloralkyl monosubstituiertes Phenyl, besonders bevorzugt durch Trifluormethyl und Trichlormethyl monosubstituiertes Phenyl wie 4-Trifluormethylphenyl und 4-Trichlormethylphenyl,
- ein- bis dreifach durch unverzweigtes oder verzweigtes $C_1$-$C_4$-Halogenalkoxy substituiertes Aryl, bevorzugt durch $C_1$-$C_2$-Fluor- und Chloralkoxy monosubstituiertes Phenyl, besonders bevorzugt durch Trifluormethoxy und Trichlormethoxy monosubstituiertes Phenyl wie Trifluormethoxyphenyl und 4-Trichlormethoxyphenyl,

X $-CH_2-$, $-OCH_2-$, $-O(CH_2)_2-$, $-O(CH_2)_3-$.

Im Gegensatz zu den meisten bisher bekannten Wirkstoffen, die als Kontakt- oder Fraßgifte die Tiere töten, lähmen oder vertreiben, wirken die meisten der Verbindungen der Formel I entwicklungsstörend auf den tierischen Organismus. Bei Insekten wird beispielsweise die Umwandlung zur Imago, die Ablage von entwicklungsfähigen Eiern und die Entwicklung von abgelegten normalen Eiern gestört und dadurch die Generationsfolge unterbrochen. Für Wirbeltiere sind die erfindungsgemäßen Wirkstoffe praktisch ungiftig. Die meisten der Verbindungen der Formel I werden überdies leicht zu Stoffen abgebaut, die in der natürlichen Umgebung vorkommen und von Mikroorganismen weiter zerlegt werden.

Die N-substituierten Azole der allgemeinen Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea ptiyocampa (Pinienprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Liniierter Blattrandkäfer), Ortiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum

(Apfelblütenstecher), Anthonomus grandis (Kapselkäfer), Ceuthorrhynchus assimilis (Kohlschotenrüßler), Ceuthorrynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);
aus der Ordnung der Zweiflügler (Diptera) beispielsweise Lycoria pectoralis, Mayetiola destructor (Hessenfliege), Basineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hysocyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ovis, Chrysomya macellaria, Chrysomya hominivorax, Lucilia cuprina, Lucilia sericata, Hypoderma lineata;
aus der Ordnung der Hautflügler (Hymenoptera) beispielsweise Athalia rose (Rübenblattwespe), Hoplocampa minuta (Pflaumensägewespe), Monomorium pharaonis (Pharaoameise), Solenopsis geminata (Feuerameise), Atta sexdens (Blattschneiderameise);
aus der Ordnung der Wanzen (Heteroptera) beispielsweise Nezara viridula (Grüne Reiswanze), Eurygaster integriceps (Asiatische Getreidewande), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);
aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nasturtii (Kreuzdornblattlaus), Cerosipha gossypii (Gurkenblattlaus), Sappaphis mali (Rosige Apfellaus), Sappaphis mala (Mehlige Birnblattlaus), Dyasphis radicola (Mehlige Apfelfaltenlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenlaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);
aus der Ordnung der Termiten (Isoptera) beispielsweise Reticulitermes lucifugus, Calotermes flavicollis, Leucotermes flavipes, Termes natalensis;
aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Küchenschabe), Blattella germanica (Deutsche Schabe), Periplaneta americana (Amerikanische Schabe), Blabera gigantes (Riesenschabe).

Zur Klasse der Arachnoidea gehören Spinntentiere (Acarina) beispielsweise Ixodes ricinus (Holzbock), Ornithodorus moubata, Amblyomma americanum, Dermacentor silvarum, Boophilus microplus, Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne incognita, Meloidogyne hapla, Meloidogyne javanica, Zysten bildende Nematoden, z.B. Globodera rostochiensis, Heterodera schacutii, Heterodera avenae, Heterodera glycinae, Heterodera triflolii, Stock- und Blattälchen, z.B. Ditylenchus dipsaci, Ditylenchus destructor, Pratylenchus neglectus, Pratylenchus penetrans, Paratylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können als Emulsionskonzentrate, Pasten oder netzbare Pulver (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkali, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Beispiele für Formulierungen sind:

I. 5 Gew.-Teile der Verbindung Nr. 1 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 3 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 20 Gew.-Teile der Verbindung Nr. 9 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

IV. 80 Gew.-Teile der Verbindung Nr. 41 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Die Wirkstoffkonzentrationen in den anwendungsfertigen Zubereitungen können in größeren Bereichen variiert werden. Im allgemeinen liegen sie zwischen 0,0001 und 10 %, vorzugsweise zwischen 0,01 und 1 %. Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,001 bis 10, insbesondere 0,1 bis 2, vorzugsweise 0,01 bis 1 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt

werden. Diese Mittel können zu den erfindungsgemäßen Mitteln im Gewichtsverhältnis 1:10 bis 10:1 zugemischt werden.

Beispielsweise können folgende Mittel zugemischt werden: 1,2-Dibrom-3-chlorpropan, 1,3-Dichlorpropen, 1,3-Dichlorpropen + 1,2-Dichlorpropan, 1,2-Dibromethan, 2-sec.-Butyl-phenyl-N-methylcarbamat, o-Chlorphenyl-N-methylcarbamat, 3-Isopropyl-5-methylphenyl-N-methylcarbamat, o-Isopropoxyphenyl-N-methylcarbamat, 3,5-Dimethyl-4-methylmercapto-phenyl-N-methylcarbamat, 4-Dimethylamino-3,5-xylyl-N-methylcarbamat, 2-(1,3-Dioxolan-2-yl)-phenyl-N-methylcarbamat, 1-Naphthyl-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-O-(methylcarbamoyl)-oxim, S-Methyl-N[(methylcarbamoyl)-oxyl]-thio-acetimidat, Methyl-N′,N′-dimethyl-N-[-(methylcarbamoyl)oxy]-1-thiooxamidat, N-(2-Methylchlorphenyl)-N′,N′-dimethylformamidin, Tetrachlorthiophen, 1-(2,6-Difluor-benzoyl)-3-(4-chlorphenyl)-harnstoff, O,O-Dimethyl-O-(p-nitrophenyl)-phosphorthioat, O,O-Diethyl-O-(p-nitrophenyl)-phosphorthioat, O-Ethyl-O(p-nitrophenyl)-phenyl-phosphonothioat, O,O-Dimethyl-O-(3-methyl-4-nitrophenyl)-phosphorthioat, O,O-Diethyl-O-(2,4-dichlorphenyl)-phosphorthioat, O-Ethyl-O-(2,4-dichlorphenyl)-phenyl-phosphonothioat, O,O-Dimethyl-O-(2,4,5-trichlorphenyl)-phosphorthioat, O-Ethyl-O-(2,4,5-trichlorphenyl)-ethyl-phosphorthioat, O,O-Dimethyl-O-(4-brom-2,5-dichlorphenyl)-phosphorthioat, O,O-Dimethyl-O-(2,5-dichlor-4-jodphenyl)-phosphorthioat, O,O-Dimethyl-O-(3-methyl-4-methylthiophenyl)-phosphorthioat, O-Ethyl-O-(3-methyl-4-methylthiophenyl)-isopropyl-phosphoramidat, O,O-Diethyl-O-(p-methylsulfinyl-phenyl)-phosphorthioat, O-Ethyl-S-phenyl-ethyl-phosphonodithioat, O,O-Diethyl-[2-chlor-1-(2,4-dichlorphenyl)-vinyl]-phosphat, O,O-Dimethyl-[2-chlor-1-(2,4,5-trichlorphenyl)]-vinylphosphat, O,O-Dimethyl-S-(1′-phenyl)-ethylacetat-phosphordithioat, Bis-(dimethylamino)-fluorphosphinoxid, Octamethyl-pyrophosphoramid, O,O,O,O-Tetraethyldithio-pyrophosphat, S-Chlormethyl-O,O-diethyl-phosphordithioat, O-Ethyl-S,S-dipropyl-phosphordithioat, O,O-Dimethyl-O-2,2-dichlorvinyl-phosphat, O,O-Dimethyl-1,2-dibrom-2,2-dichlorethylphosphat, O,O-Dimethyl-2,2,2-trichlor-1-hydroxy-ethylphosphonat, O,O-Dimethyl-S-[1,2-biscarbethoxy-ethyl-(1)]-phosphordithioat, O,O-Dimethyl-O-(1-methyl-2-carbmethoxy-vinyl)-phosphat, O,O-Dimethyl-S-(N-methyl-carbamoyl-methyl)-phosphordithioat, O,O-Dimethyl-S-(N-methylcarbamoyl-methyl)-phosphorthioat, O,O-Dimethyl-S-(N-methoxyethyl-carbamoylmethyl)-phosphordithioat, O,O-Dimethyl-S-(N-formyl-N-methyl-carbamoyl-methyl)-phosphordithioat, O,O-Dimethyl-O-[1-methyl-2-(methyl-carbamoyl)-vinyl]-phosphat, O,O-Dimethyl-O-[(1-methyl-2-dimethylcarbamoyl)-vinyl]-phosphat, O,O-Dimethyl-O-[(1-methyl-2-chlor-2-diethylcarbamoyl)-vinyl]-phosphat, O,O-Diethyl-S-(ethylthiomethyl)-phosphordithioat, O,O-Diethyl-S-[(p-chlorphenylthio)-methyl]-phosphordithioat, O,O-Dimethyl-S-(2-ethylthioethyl)-phosphorthioat, O,O-Dimethyl-S-(2-ethylthioethyl)-phosphordithioat, O,O-Dimethyl-S-(2-ethylsulfinyl-ethyl)-phosphorthioat, O,O-Diethyl-S-(2-ethylthio-ethyl)-phosphordithioat, O,O-Diethyl-S-(2-ethylsulfinyl-ethyl)-phosphorthioat, O,O-Diethyl-thiophosphoryliminophenyl)-acetonitril, O,O-Diethyl-S-(2-chlor-1-phthalimidoethyl)-phosphordithioat, O,O-Diethyl-S-[6-chlor-benzoxazolon-(2)-yl(3)]-methyldithiophosphat, O,O-Dimethyl-S-[2-methoxy-1,3,4-thiadiazol-5-[4H]-onyl-(4)-methyl]-phosphordithioat, O,O-Diethyl-O-[3,5,6-trichlor-pyridyl-(2)]-phosphorthioat, O,O-Diethyl-O-(2-pyrazinyl)-phosphorthioat, O,O-Diethyl-O-[2-isopropyl-4-methyl-pyrimidinyl(6)]-phosphorthioat, O,O-Diethyl-O-[2-(diethylamino)-6-methyl-4-pyrimidinyl]-thionophosphat, O,O-Dimethyl-S-(4-oxo-1,2,3-benzotriazin-3-[4H]-yl-methyl)-phosphordithioat, O,O-Dimethyl-S-[(4,6-diamino-1,3,5-triazin-2-yl)-methyl]-phosphordithioat, O,O-Diethyl-(1-phenyl-1,2,4-triazol-3-yl)-thionophosphat, O,S-Dimethyl-phosphor-amidothioat, O,S-Dimethyl-N-acetyl-phosphoramidothioat, alpha-Hexachlorcyclohexan, 1,1-Di-(p-methoxyphenyl)-2,2,2-trichlor-ethan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on-(1)-yl-(4)-DL-cis,-trans-chrysanthemat, 5-Benzyl-furyl-(3)-methyl-DL-cis,-trans-chrysanthemat, 3-Phenoxybenzyl(±)-cis,-trans-2,2-dimethyl-3-(2,2-dichlorvinyl)-cyclopropancarboxylat, alpha-Cyano-3-phenoxybenzyl(±)-cis,-trans-2,2-dimethyl-3-(2,2-dichlorvinyl)-cyclopropancarboxylat, (s)-alpha-Cyano-3-phenoxybenzyl-cis(1R,3R)-2,2-dimethyl-3-(2,2-dibromvinyl)-cyclopropancarboxylat, 3,4,5,6-Tetrahydrophthalimidoethyl-DL-cis,-trans-chrysanthemat, 2-Methyl-5-(2-propinyl)-3-furylmethyl-chrysanthemat, (alpha-Cyano-3-phenoxybenzyl)-alpha-isopropyl-4-chlorphenylacetat.

## Herstellungsbeispiele

### Beispiel 1

N-[(p-Phenoxy-phenoxy)-methyl]-4,5-dichlorimidazol (Verbindung 1)

Man löst 94 g 1-Hydroxymethyl-4,5-dichlorimidazol in 550 ml Tetrachlorkohlenstoff, fügt 1 ml Dimethylformamid hinzu und läßt unter Rühren 100,5 g Thionylchlorid eintropfen. Die Mischung wird bis zur Beendigung der Gasentwicklung unter Rückfluß erhitzt, anschließend das Solvens i.Vak. entfernt und der

Rückstand mit 750 ml Methylenchlorid und 250 ml Wasser versetzt. Die wäßrige Phase wird mittels 5-%iger Natronlauge unter intensivem Rühren auf pH 7,5 gebracht, die organische Phase abgetrennt, mit Wasser gewaschen und über Natriumsulfat getrocknet. Der nach dem Entfernen des Lösungsmittels verbleibende Rückstand wird bei 92 bis 95 ° C/0,1 mbar destilliert. Man erhält 80,5 g (77 %) 1-Chlormethyl-4,5-dichlorimidazol; Fp.: 40 bis 41 ° C.

9,3 g 4-Phenoxyphenol werden in 60 ml trockenem Dimethylformamid gelöst. Man fügt 1,58 g 80 %iges Natriumhydrid hinzu und erwärmt die Mischung bis zur Beendigung der Wasserstoffentwicklung auf 60 ° C (ca. 1 Stunde). Anschließend tropft man eine Lösung von 9,28 g N-(1-Chlormethyl)-4,5-dichlorimidazol in 30 ml Dimethylformamid hinzu. Man läßt über Nacht bei Raumtemperatur rühren, gießt den Ansatz in 500 ml Wasser und extrahiert dreimal mit Methyl-tert. butylether. Die vereinigten organischen Phasen werden dreimal mit Wasser gewaschen, über Natriumsulfat getrocknet und i.Vak. vom Lösungsmittel befreit. Der feste Rückstand läßt sich aus n-Pentan/Ether = 6:1 umkristallisieren und man erhält N-[(p-Phenoxy-phenoxy)-methyl]-4,5-dichlorimidazol; Fp.: 82 bis 83 ° C.

| $C_{16}H_{12}Cl_2N_2O_2$ (335) | | | |
|---|---|---|---|
| ber.: | C 57,4 | H 3,6 | N 8,4 |
| gef.: | C 57,7 | H 3,7 | N 8,3 |

Infrarotabsorptionen ($cm^{-1}$): 1478, 1403, 1235, 1209, 1047, 854

Beispiel 2

N-[p-(3-Chlorphenoxy)-benzyl]-4,5-dimethylimidazol (Verbindung 2)

Unter intensivem Rühren trägt man 1,91 g 80 %-iges Natriumhydrid in eine Lösung von 6,04 g 4,5-Dimethylimidazol in 80 ml Tetrahydrofuran ein. Nach Beendigung der exothermen Reaktion wird die Mischung noch für 3 Stunden auf 60 ° C gehalten und nach dem Abkühlen mit einer Lösung von 17,85 g p-(3-Chlorphenoxy)-benzylbromid in 20 ml Tetrahydrofuran versetzt. Der Reaktionsansatz wird 12 Stunden bei 20 ° C gerührt, anschließend in ca. 750 ml Eiswasser gegossen und dreimal mit Methyl-tert.-butylether ausgeschüttelt. Den Extrakt wäscht man je zweimal mit 5 %iger Natronlauge und Wasser. Nach dem Trocknen über Magnesiumsulfat wird das Lösungsmittel i.Vak. entfernt und der Rückstand über Kieselgel 60 mit Aceton/Essigester = 2:1 chromatographiert. Man erhält 12,1 g (65 %) N-[p-(3-Chlorphenoxy)-benzyl]-4,5-dimethylimidazol als harzähnliche Flüssigkeit, die beim Stehen langsam durchkristallisiert.

| $C_{18}H_{17}ClN_2O$ (312,5) | | | |
|---|---|---|---|
| ber.: | C 69,1 | H 5,5 | N 9,0 |
| gef.: | C 69,1 | H 5,5 | N 8,9 |

Infrarotabsorptionen ($cm^{-1}$): 1505, 1474, 1306, 1226, 901

Beispiel 3

N-[(p-Phenoxyphenoxy)-ethyl]-4,5-dichlorimidazol (Verbindung 3)

Man löst 23,0 g 2-(4-Phenoxyphenoxy)-ethanol und 22,9 g p-Toluolsulfonsäurechlorid in 100 ml Methylenchlorid und läßt zwischen 10 und 15 ° C 18,96 g Pyridin hinzutropfen. Die Mischung wird 30 Minuten bei Raumtemperatur und 4 Stunden unter Rückfluß gerührt. Danach wird das Lösungsmittel i.Vak. entfernt, der Rückstand in 150 ml Pyridin gelöst und in 600 ml Eiswasser gegossen. Der ausgefallene Feststoff wird abfiltriert, getrocknet und aus Cyclohexan/Methyl-tert.-buthylether = 5:1 umkristallisiert. Man erhält 28,2 g (73 %) p-Toluolsulfonsäure-2-[(p-phenoxyphenoxy)-ethyl]-ester; Fp. 53 bis 55 ° C.

Eine Mischung aus 8,43 g 4,5-Dichlorimidazol, 80 ml Dimethylformamid und 1,83 g 80 %-igem Natriumhydrid wird unter Rühren für drei Stunden auf 60 ° C erwärmt. Anschließend wird eine Lösung von 23,04 g p-Toluolsulfonsäure-2-[(4-phenoxy)phenoxy]-ethylester in 40 ml Dimethylformamid tropfenweise hinzugefügt. Man erhitzt 12 Stunden auf 100 ° C, gießt nach dem Abkühlen in 50 ml Eiswasser, extrahiert mehrfach mit Essigester und wäscht die vereinigten Extrakte nacheinander mit 5 %-iger Natronlauge und

Wasser. Nach dem Trocknen über Natriumsulfat, Entfernen des Lösungsmittels i.Vak. und Umkristallisieren aus Methyl-tert.-butylether erhält man 16,9 g (81 %) N-[(p-Phenoxyphenoxy)-ethyl]-4,5-dichlorimidazol; Fp.: 89 bis 91 ° C.

| $C_{17}Cl_2N_2O_2$ (349) | | | |
|---|---|---|---|
| ber.: | C 58,5 | H 4,0 | N 8,0 |
| gef.: | C 58,2 | H 4,1 | N 7,9 |

Infrarotabsorptionen ($cm^{-1}$): 1489, 1462, 1255, 1214, 1190, 1063, 845

Beispiel 4

N-[(p-Phenoxyphenoxy)-n-propyl]-imidazol (Verbindung 4)

Zu einer Lösung von 9,5 g Phosphortribromid in 65 ml Tetrachlormethan fügt man portionsweise 24,4 g 3-(4-Phenoxyphenoxy)-propan-1-ol. Nach Abklingen der exothermen Reaktion wird die Mischung für 4 Stunden auf 40 ° C erwärmt. Nach dem Erkalten entfernt man das Lösungsmittel i.Vak., löst den Rückstand in 300 ml Methyl-tert.-butylether, wäscht die organische Phase gründlich mit Wasser und trocknet über Natriumsulfat. Das nach Entfernen des Solvens verbleibende Rohprodukt läßt sich durch Kieselgel-Filtration mit Toluol/Methyl-tert.-butylether = 2:1 reinigen. Man erhält 20,8 g (67 %) 3-(p-Phenoxyphenoxy)-1-brom-propan; Fp.: 54 bis 55 ° C.

Eine Lösung von 4,25 g Imidazol in 70 ml Dimethylformamid wird portionsweise mit 1,98 g 80 %-igem Natriumhydrid versetzt. Nach dem Abklingen der Wasserstoffentwicklung läßt man noch 3 Stunden bei 60 ° C nachrühren und fügt anschließend eine Lösung von 18,42 g 3-[4-(Phenoxy)phenoxy]-1-brom-propan in 40 ml Dimethylformamid hinzu. Die Mischung wird für 8 Stunden auf 80 ° C erwärmt und danach in 2,5 l Eiswasser gegossen. Der abgeschiedene Feststoff wird abfiltriert im Exsikkator über Calciumchlorid getrocknet und durch Filtration über Kieselgel gereinigt. Man erhält 14,5 g (82 %) N-[(p-Phenoxyphenoxy)-n-propyl]-imidazol; Fp.: 54,5 bis 55,5 ° C.

| $C_{18}H_{18}N_2O_2$ (294) | | | |
|---|---|---|---|
| ber.: | C 73,4 | H 6,2 | N 9,5 |
| gef.: | C 73,1 | H 6,3 | N 9,4 |

Infrarotabsorptionen ($cm^{-1}$): 1489, 1224, 1196, 1166, 1075, 849

Beispiel 5

1-{[p-(3-Methyl)-phenoxy-phenoxy]-methyl}-1,2,4-triazol (Verbindung Nr. 135)

Zu 10 g p-(3-Methylphenoxy)-phenol und 20,7 g Kaliumcarbonat in 60 ml trockenem Dimethylformamid gibt man bei Raumtemperatur (ca. 20 ° C) 7,7 g 1-Chlormethyl-1,2,4-triazol-hydrochlorid. Anschließend rührt man 6 Stunden bei 70 ° C und über Nacht bei Raumtemperatur (ca. 20 ° C) nach. Zur Aufarbeitung gießt man in 300 ml Wasser und extrahiert dreimal mit Methyl-tert.-butylether. Die vereinigten organischen Phasen werden dreimal mit Wasser gewaschen, über Natriumsulfat getrocknet und eingeengt. Der Rückstand wird durch Chromatographie an Kieselgel, mit Toluol/Methyl-tert.-butylether = 2/1 als Eluent, gereinigt. Man erhält 5,1 g 1-{[4-(3-Methyl)-phenoxy-phenoxy]-methyl}-1,2,4-triazol vom Fp.: 52-54 ° C.

Beispiel 6

2-Chlor-1-[(p-Phenoxy-phenoxy)-methyl]-1,3,4-triazol (Verbindung Nr. 177)

5,6 g p-Phenoxyphenol und 4,2 g Kaliumcarbonat werden in 50 ml trockenem Dimethylformamid 1 Stunde auf 70 ° C erwärmt. Anschließend gibt man 5,6 g 2-Chlor-1-chlormethyl-1,3,4-triazol zu und rührt 6 Stunden bei 70 ° C und über Nacht bei Raumtemperatur (ca. 20 ° C) nach. Zur Aufarbeitung gießt man auf 500 ml Wasser und extrahiert dreimal mit Essigester. Die vereinigten organischen Phasen werden über

Magnesiumsulfat getrocknet und eingeengt. Der Rückstand wird durch Chromatographie an Kieselgel gereinigt mit n-Hexan + Essigester = 2/1 als Eluent. Man erhält 2,8 g 2-Chlor-1-[(p-Phenoxy-phenoxy)-methyl]-1,3,4-triazol als Öl.

300 MHz NMR in $CDCl_3$ δ [ppm]: 5,95 (2H), 6,90-7,15 (7H), 7,25-7,38 (2H), 8,15 (1H).

Entsprechend dieser Herstellvorschriften können die in den folgenden Tabellen 1 bis 4 beschriebenen Verbindungen Ia-Id hergestellt werden.

Tabelle 1

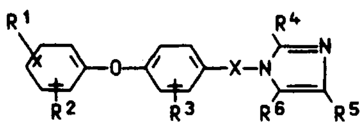

(Ia)

| Verbindung Nr. | $R^1$ | $R^2$ | $R^3$ | X | $R^4$ | $R^5=R^6$ | phys. Daten [IR-Absorptionen ($cm^{-1}$)] |
|---|---|---|---|---|---|---|---|
| 1 | H | H | H | $OCH_2$ | H | Cl | 1478, 1403, 1235, 1209 |
| 2 | 3-Cl | H | H | $CH_2$ | H | $CH_3$ | 1505, 1474, 1306, 1226 |
| 3 | H | H | H | $O(CH_2)_2$ | H | Cl | 1489, 1462, 1255, 1214 |
| 4 | H | H | H | $O(CH_2)_3$ | H | H | 1489, 1224, 1196, 1166 |
| 5 | 4-F | H | H | $CH_2$ | H | Cl | 1250, 1212, 1193, 1180 |
| 6 | $4-OCH_3$ | H | H | $CH_2$ | H | $CH_3$ | 1443, 1228, 1034 |
| 7 | 4-Cl | H | H | $CH_2$ | H | $CH_3$ | 1240, 1167, 1089 |
| 8 | 4-Cl | H | H | $CH_2$ | H | Cl | 1244, 1168, 1098, 1018 |
| 9 | 3-Cl | H | H | $CH_2$ | H | Cl | 1471, 1238, 1169 |
| 10 | H | H | H | $CH_2$ | H | Cl | 1242, 1169, 983 |
| 11 | H | H | H | $CH_2$ | H | $CH_3$ | 1447, 1237, 1167 |
| 12 | $4-NO_2$ | H | H | $CH_2$ | H | $CH_3$ | 1336, 1248, 1201, 1189 |
| 13 | $4-NO_2$ | H | H | $CH_2$ | H | Cl | 1338, 1247, 1202, 1167 |
| 14 | 4-F | H | H | $CH_2$ | H | $CH_3$ | 1253, 1213, |
| 15 | $4-C_2H_5$ | H | H | $CH_2$ | H | Cl | 1245, 1170, 982 |
| 16 | $4-OCH_3$ | H | H | $CH_2$ | H | Cl | 1250, 1169, 1033 |
| 17 | $4-CH_3$ | H | H | $CH_2$ | H | Cl | 1248, 1172 |
| 18 | $4-CH_3$ | H | H | $CH_2$ | H | $CH_3$ | 1241, 1171 |

| Verbindung Nr. | $R^1$ | $R^2$ | $R^3$ | X | $R^4$ | $R^5=R^6$ | phys. Daten [IR-Absorptionen (cm$^{-1}$)] |
|---|---|---|---|---|---|---|---|
| 19 | 4-$C_2H_5$ | H | H | $CH_2$ | H | $CH_3$ | 1447, 1238, 1169 |
| 20 | H | H | H | $CH_2$ | H | Cl | |
| 21 | 3-$CF_3$ | H | H | $CH_2$ | H | Cl | |
| 22 | 3-F | 4-F | H | $CH_2$ | H | Cl | |
| 23 | 4-$CF_3$ | H | H | $CH_2$ | H | Cl | |
| 24 | H | H | H | $CH_2$ | Br | Cl | |
| 25 | H | H | H | $CH_2$ | Cl | Cl | |
| 26 | H | H | H | $O(CH_2)_2$ | H | $CH_3$ | 1447, 1254, 1220, 1059 |
| 27 | H | H | H | $O(CH_2)_2$ | H | H | 1460, 1231, 1218, 1108 |
| 28 | 3-F | H | H | $O(CH_2)_2$ | H | Cl | |
| 29 | 3-Cl | H | H | $O(CH_2)_2$ | H | Cl | |
| 30 | 3-$CF_3$ | H | H | $O(CH_2)_2$ | H | Cl | |
| 31 | 3-F | 4-F | H | $O(CH_2)_2$ | H | Cl | |
| 32 | 4-$CF_3$ | H | H | $O(CH_2)_2$ | H | Cl | |
| 33 | 3-$CH_3$ | H | H | $O(CH_2)_2$ | H | Cl | |
| 34 | H | H | H | $O(CH_2)_2$ | Cl | Cl | |
| 35 | 3-Cl | H | H | $O(CH_2)_2$ | Cl | Cl | |
| 36 | H | H | H | $O(CH_2)_2$ | Br | Cl | |
| 37 | 3-Cl | H | H | $O(CH_2)_2$ | Br | Cl | |
| 38 | 3-F | H | H | $O(CH_2)_2$ | Br | Cl | |
| 39 | 3-$CF_3$ | H | H | $O(CH_2)_2$ | Br | Cl | |
| 40 | 3-F | 4-F | H | $O(CH_2)_2$ | Br | Cl | |
| 41 | H | H | H | $O(CH_2)_3$ | H | Cl | 1258, 1250, 1219, 1191 |
| 42 | H | H | H | $O(CH_2)_2$ | H | $CH_3$ | 1473, 1220, 1186 |
| 43 | 4-F | H | H | $O(CH_2)_2$ | H | Cl | |
| 44 | 3-F | H | H | $OCH_2$ | H | Cl | 1483, 1205, 1116, 1035 |
| 45 | 3-Cl | H | H | $OCH_2$ | H | Cl | 1472, 1240, 1212, 1186 |
| 46 | 3-$CF_3$ | H | H | $OCH_2$ | H | Cl | 1329, 1213, 1185, 1125 |
| 47 | 3-$CH_3$ | H | H | $OCH_2$ | H | Cl | 1501, 1255, 1244, 1203 |
| 48 | 3-F | 4-F | H | $OCH_2$ | H | Cl | 1250, 1225, 1203, 1187 |

| Verbindung Nr. | $R^1$ | $R^2$ | $R^3$ | X | $R^4$ | $R^5$=$R^6$ | phys. Daten [IR-Absorptionen ($cm^{-1}$)] |
|---|---|---|---|---|---|---|---|
| 49 | 4-F | H | H | $OCH_2$ | H | Cl | 1496, 1403, 1236, 1230 |
| 50 | 4-$CF_3$ | H | H | $OCH_2$ | H | Cl | |
| 51 | H | H | H | $OCH_2$ | Br | Cl | |
| 52 | 4-Cl | H | H | $OCH_2$ | Br | Cl | |
| 53 | 3-F | H | H | $OCH_2$ | Br | Cl | |
| 54 | 3-$CF_3$ | H | H | $OCH_2$ | Br | Cl | |
| 55 | 3-$CH_3$ | H | H | $OCH_2$ | Br | Cl | |
| 56 | 4-F | H | H | $OCH_2$ | Br | Cl | |
| 57 | 4-$CF_3$ | H | H | $OCH_2$ | Br | Cl | |
| 58 | 4-Cl | H | H | $OCH_2$ | Br | Cl | |
| 59 | 3-F | 4-F | H | $OCH_2$ | Br | Cl | |
| 60 | H | H | H | $OCH_2$ | Cl | Cl | |
| 61 | 3-Cl | H | H | $OCH_2$ | Cl | Cl | |
| 62 | 3-F | H | H | $OCH_2$ | Cl | Cl | |
| 63 | 3-$CF_3$ | H | H | $OCH_2$ | Cl | Cl | |
| 64 | 4-F | H | H | $OCH_2$ | Cl | Cl | |
| 65 | 4-$CF_3$ | H | H | $OCH_2$ | Cl | Cl | |
| 66 | 4-Cl | H | H | $OCH_2$ | Cl | Cl | |
| 67 | 3-Cl | H | H | $O(CH_2)_3$ | H | Cl | |
| 68 | 4-F | H | H | $O(CH_2)_3$ | H | Cl | |
| 69 | 4-$CF_3$ | H | H | $O(CH_2)_3$ | H | Cl | |
| 70 | 4-$CH_3$ | H | H | $O(CH_2)_3$ | H | Cl | |
| 71 | 3-F | 4-F | H | $O(CH_2)_3$ | H | Cl | |
| 72 | 4-F | H | H | $O(CH_2)_3$ | H | Cl | |
| 73 | 4-Cl | H | H | $O(CH_2)_3$ | H | Cl | |
| 74 | H | H | H | $O(CH_2)_3$ | Cl | Cl | |
| 75 | H | H | H | $O(CH_2)_3$ | Br | Cl | |
| 76 | 3-Cl | H | H | $O(CH_2)_3$ | Cl | Cl | |
| 77 | 3-$C(CH_3)_3$ | H | H | $OCH_2$ | H | Cl | 1405, 1235, 1214, 1040 |
| 78 | 3-$OCH_3$ | H | H | $OCH_2$ | H | Cl | 1488, 1243, 1204, 1138 |
| 79 | 3-$C_2H_5$ | H | H | $OCH_2$ | H | Cl | 1485, 1447, 1244, 1224 |
| 80 | 3-Br | H | H | $OCH_2$ | H | Cl | 1490, 1470, 1402, 1237 |
| 81 | 4-Cl | H | H | $OCH_2$ | H | Cl | 1502, 1489, 1253, 1245 |

| Verbindung Nr. | $R^1$ | $R^2$ | $R^3$ | X | $R^4$ | $R^5$=$R^6$ | phys. Daten [IR-Absorptionen ($cm^{-1}$)] |
|---|---|---|---|---|---|---|---|
| 82 | 4-Br | H | H | $OCH_2$ | H | Cl | 1486, 1244, 1206, 1183 |
| 83 | 4-$CH_3$ | H | H | $OCH_2$ | H | Cl | 1501, 1257, 1245, 1204 |
| 84 | 4-$C_2H_5$ | H | H | $OCH_2$ | H | Cl | 1498, 1242, 1206, 1186 |
| 85 | 4-F | H | H | $OCH_2$ | H | Cl | 1481, 1244, 1221, 1203 |
| 86 | 3-Cl | 4-Cl | H | $OCH_2$ | H | Cl | 1502, 1468, 1255, 1244 |
| 87 | 4-tert-$C_4H_9$ | H | H | $OCH_2$ | H | Cl | 1501, 1481, 1406, 1231 |
| 88 | 4-$OCH_3$ | H | H | $OCH_2$ | H | Cl | 1498, 1489, 1405, 1236 |
| 89 | 4-$OC_2H_5$ | H | H | $OCH_2$ | H | H | 1498, 1490, 1405, 1239 |
| 90 | H | H | H | $OCH_2$ | H | H | 1487, 1403, 1213, 1019 |
| 91 | 3-F | H | H | $OCH_2$ | H | H | 1485, 1271, 1291, 1204 |
| 92 | 3-Cl | H | H | $OCH_2$ | H | H | 1485, 1227, 1214, 1191 |
| 93 | 3-Br | H | H | $OCH_2$ | H | H | 1470, 1226, 1213, 1073 |
| 94 | 3-$CH_3$ | H | H | $OCH_2$ | H | H | 1487, 1255, 1221, 1205 |
| 95 | H | H | H | $OCH_2$ | H | $CH_3$ | 1489, 1406, 1208, 1165 |
| 96 | 3-F | H | H | $OCH_2$ | H | $CH_3$ | 1485, 1271, 1231, 1204 |
| 97 | 3-Cl | H | H | $OCH_2$ | H | $CH_3$ | 1497, 1472, 1231, 1212 |
| 98 | 3-Br | H | H | $OCH_2$ | H | $CH_3$ | 1470, 1424, 1224, 1208 |
| 99 | 3-$CH_3$ | H | H | $OCH_2$ | H | $CH_3$ | 1499, 1403, 1233, 1211 |
| 100 | 3-tert-$C_4H_9$ | H | H | $OCH_2$ | H | $CH_3$ | 1403, 1271, 1235, 1214 |

Tabelle 2

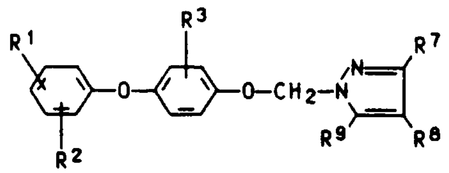

(Ib)

| Verbindung Nr. | $R^1$ | $R^2$ | $R^3$ | $R^7$ | $R^8$ | $R^9$ | Phys. Daten |
|---|---|---|---|---|---|---|---|
| 101 | H | H | H | H | H | H | |
| 102 | 3-F | H | H | H | H | H | |
| 103 | 3-Cl | H | H | H | H | H | |
| 104 | 3-Br | H | H | H | H | H | |
| 105 | 3-$CF_3$ | H | H | H | H | H | |
| 106 | 3-$CH_3$ | H | H | H | H | H | |
| 107 | 3-$C_2H_5$ | H | H | H | H | H | |
| 108 | 3-$OCH_3$ | H | H | H | H | H | |
| 109 | 3-Cl | 4-F | H | H | H | H | |
| 110 | H | H | H | $CH_3$ | H | H | |
| 111 | 3-F | H | H | $CH_3$ | H | H | |
| 112 | 3-Cl | H | H | $CH_3$ | H | H | |
| 113 | 3-Br | H | H | $CH_3$ | H | H | |
| 114 | 3-$CF_3$ | H | H | $CH_3$ | H | H | |
| 115 | 3-$CH_3$ | H | H | $CH_3$ | H | H | |
| 116 | 3-$C_2H_5$ | H | H | $CH_3$ | H | H | |
| 117 | 3-$OCH_3$ | H | H | $CH_3$ | H | H | |
| 118 | 3-Cl | 4-F | H | $CH_3$ | H | H | |
| 119 | H | H | H | $CH_3$ | H | $CH_3$ | 300 MHz-$^1$H-NMR in $CDCl_3$ [ppm]: 5,81 (s) |
| 120 | 3-F | H | H | $CH_3$ | H | $CH_3$ | |
| 121 | 3-Cl | H | H | $CH_3$ | H | $CH_3$ | |
| 122 | 3-Br | H | H | $CH_3$ | H | $CH_3$ | |
| 123 | 3-$CF_3$ | H | H | $CH_3$ | H | $CH_3$ | |
| 124 | 3-$CH_3$ | H | H | $CH_3$ | H | $CH_3$ | |
| 125 | 3-$C_2H_5$ | H | H | $CH_3$ | H | $CH_3$ | |
| 126 | 3-$OCH_3$ | H | H | $CH_3$ | H | $CH_3$ | |
| 127 | 3-Cl | 4-F | H | $CH_3$ | H | $CH_3$ | |

Tabelle 3

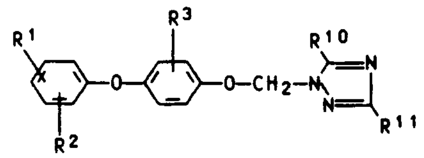

(Ic)

| Verbindung Nr. | $R^1$ | $R^2$ | $R^3$ | $R^{10}$ | $R^{11}$ | Phys. Daten |
|---|---|---|---|---|---|---|
| 128 | H | H | H | H | H | Fp.: 66-67°C |
| 129 | H | H | 3-F | H | H | |
| 130 | H | H | 3-Cl | H | H | |
| 131 | 3-F | H | H | H | H | Fp.: 39-41°C |
| 132 | 3-Cl | H | H | H | H | IR-Absorptionen ($cm^{-1}$): 1502, 1472, 1274, 1232 |
| 133 | 3-Br | H | H | H | H | IR-Absorptionen ($cm^{-1}$): 1502, 1469, 1273, 1230, 1207 |
| 134 | $3-CF_3$ | H | H | H | H | IR-Absorptionen ($cm^{-1}$): 1503, 1450, 1329, 1276 |
| 135 | $3-CH_3$ | H | H | H | H | Fp.: 52-54°C |
| 136 | $3-C_2H_5$ | H | H | H | H | Fp.: 39-41°C |
| 137 | 3-tert.-Butyl | H | H | H | | IR-Absorptionen ($cm^{-1}$): 1502, 1487, 1430, 1273, 1231 |
| 138 | $3-OCH_3$ | H | H | H | | IR-Absorptionen ($cm^{-1}$): 1502, 1489, 1274, 1223, 1206 |
| 139 | 3-F | 4-F | H | H | H | Fp.: 68-70°C |
| 140 | 3-Cl | 4-F | H | H | H | Fp.: 74-75°C |
| 141 | H | H | 3-F | H | H | |
| 142 | H | H | 3-Cl | H | H | |
| 143 | 3-F | H | 3-Cl | H | H | |
| 144 | 3-F | H | 3-F | H | H | |
| 145 | 3-Cl | H | 3-F | H | H | |
| 146 | 3-Cl | H | 3-Cl | H | H | IR-Absorptionen ($cm^{-1}$): 1586, 1508, 1491, 1473, 1274 |
| 147 | 3-Br | H | 3-F | H | H | |
| 148 | 3-Br | H | 3-Cl | H | H | IR-Absorptionen ($cm^{-1}$): 1583, 1490, 1470, 1273, 1260 |
| 149 | $3-CF_3$ | H | 3-F | H | H | |

Tabelle 3 - Forts.

| Verbindung Nr. | $R^1$ | $R^2$ | $R^3$ | $R^{10}$ | $R^{11}$ | Phys. Daten |
|---|---|---|---|---|---|---|
| 150 | 3-$CF_3$ | H | 3-Cl | H | H | IR-Absorptionen ($cm^{-1}$): 1509, 1491, 1451, 1329, 1275 |
| 151 | 3-$CH_3$ | H | 3-F | H | H | |
| 152 | 3-$CH_3$ | H | 3-Cl | H | H | |
| 153 | 3-$C_2H_5$ | H | 3-F | H | H | |
| 154 | 3-$C_2H_5$ | H | 3-Cl | H | H | |
| 155 | 3-$OCH_3$ | H | 3-F | H | H | |
| 156 | 3-$OCH_3$ | H | 3-Cl | H | H | |
| 157 | 3-Cl | 4-F | 3-F | H | H | |
| 158 | 3-Cl | 4-F | 3-Cl | H | H | |
| 159 | H | H | H | $CH_3$ | $CH_3$ | |
| 160 | 3-F | H | H | $CH_3$ | $CH_3$ | |
| 161 | 3-Cl | H | H | $CH_3$ | $CH_3$ | |
| 162 | 3-Br | H | H | $CH_3$ | $CH_3$ | |
| 163 | 3-$CF_3$ | H | H | $CH_3$ | $CH_3$ | |
| 164 | 3-$CH_3$ | H | H | $CH_3$ | $CH_3$ | |
| 165 | 3-$C_2H_5$ | H | H | $CH_3$ | $CH_3$ | |
| 166 | 3-$OCH_3$ | H | H | $CH_3$ | $CH_3$ | |
| 167 | 3-Cl | 4-F | H | $CH_3$ | $CH_3$ | |

Tabelle 4

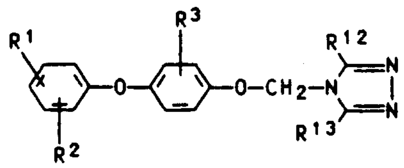

(Id)

| Verbindung Nr. | $R^1$ | $R^2$ | $R^3$ | $R^{12}$ | $R^{13}$ | Phys. Daten |
|---|---|---|---|---|---|---|
| 168 | H | H | H | H | H | |
| 169 | 3-F | H | H | H | H | |
| 170 | 3-Cl | H | H | H | H | |
| 171 | 3-Br | H | H | H | H | |
| 172 | $3-CF_3$ | H | H | H | H | |
| 173 | $3-CH_3$ | H | H | H | H | |
| 174 | $3-C_2H_5$ | H | H | H | H | |
| 175 | $3-OCH_3$ | H | H | H | H | |
| 176 | 3-Cl | 4-F | H | H | H | |
| 177 | H | H | H | H | Cl | 300 MHz-$^1$H-NMR in $CDCl_3$ [ppm]: 5,95 (s) |
| 178 | 3-F | H | H | H | Cl | |
| 179 | 3-Cl | H | H | H | Cl | |
| 180 | 3-Br | H | H | H | Cl | 300 MHz-$^1$H-NMR in $CDCl_3$ [ppm]: 8,21 (s) |
| 181 | $3-CF_3$ | H | H | H | Cl | 300 MHz-$^1$H-NMR in $CDCl_3$ [ppm]: 5,97 (s) |
| 182 | $3-CH_3$ | H | H | H | Cl | Fp.: 59-62°C |
| 183 | $3-C_2H_5$ | H | H | H | Cl | |
| 184 | $3-OCH_3$ | H | H | H | Cl | |
| 185 | 3-Cl | 4-F | H | H | Cl | |
| 186 | H | H | H | $CH_3$ | $CH_3$ | |
| 187 | 3-F | H | H | $CH_3$ | $CH_3$ | |
| 188 | 3-Cl | H | H | $CH_3$ | $CH_3$ | |
| 189 | 3-Br | H | H | $CH_3$ | $CH_3$ | |
| 190 | $3-CF_3$ | H | H | $CH_3$ | $CH_3$ | |
| 191 | $3-CH_3$ | H | H | $CH_3$ | $CH_3$ | |
| 192 | $3-C_2H_5$ | H | H | $CH_3$ | $CH_3$ | |
| 193 | $3-OCH_3$ | H | H | $CH_3$ | $CH_3$ | |
| 194 | 3-Cl | 4-F | H | $CH_3$ | $CH_3$ | |

Anwendungsbeispiele

In den folgenden Beispielen wurden die erfindungsgemäßen Verbindungen bzw. sie enthaltende Mittel hinsichtlich ihrer Wirkung auf Schädlinge mit den nachstehenden Verbindungen des Standes der Technik verglichen.

A: 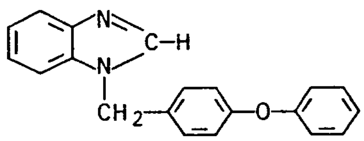 bekannt aus EP-A-132 606 als Verbindung Nr. 1

B: 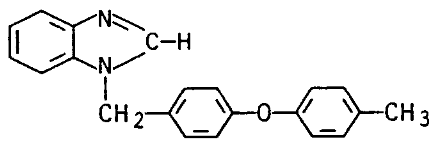 bekannt aus EP-A-132 606 als Verbindung Nr. 8

C: 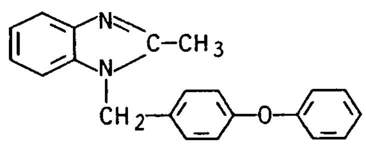 bekannt aus EP-A-132 606 als Verbindung Nr. 29

Die Konzentrationen, bei denen die untersuchten Verbindungen eine 100 %ige Mortalität bzw. Hemmung bewirken, sind die jeweiligen Minimalkonzentrationen. Bei jeder Konzentration wurden mindestens 2 Versuche angesetzt.

Beispiel A

Dysdercus intermedius (Baumwollwanze) ; Zuchtversuch

Der Versuch erfolgt in 1 l-Gläser auf 200 g sterilem Quarzsand (Korngröße 0-3 mm). Dieser Sand wird vor Versuchsbeginn mit 20 ml der wäßrigen Wirkstoffaufbereitung angegossen. Darauf belegt man die Gläser mit 10 Larven des vierten Larvenstadiums. Als Futter bietet man gequollene Baumwollsaat, die wöchentlich gewechselt wird. Ebenfalls wöchentlich wird der Sand mit reinem Wasser angefeuchtet. Die Versuchstemperatur beträgt 25 bis 27 °C. Die Beobachtungszeit erstreckt sich bis zur Adulthäutung. Die Probe gilt als wirksam, wenn die Tiere bei Versuchsende entweder tot sind oder Riesenlarven oder Zwischentypen (Adultoide) darstellen oder stärkere morphologische Defekte aufweisen.

Bei diesem Versuch wurde mit den Verbindungen Nr. 128, 131, 132, 134 und 177 eine 100 %ige Mortalitätsrate mit ≦ 10 ppm an Wirkstoff erzielt. Die Vergleichsverbindungen A, B und C zeigten bei 10 ppm keine Wirkung.

Beispiel B

Prodenia litura (ägypt. Baumwollwurm) ;, ovizide Wirkung

Eiablagen von Prodenia litura, die nicht älter als 24 Stunden sind, werden aus dem Pergamentpapier ausgeschnitten. Darauf werden dann 0,5 ml der wäßrigen Aufbereitung der Wirkstoffe aufpipettiert. Die behandelten Eier placiert man in Kunststoffpaletten, die mit Filterpapier ausgelegt wurden. Anschließend deckt man mit einer Glasplatte ab. Die Bonitur erfolgt nach ca. 5 Tagen, wenn in der Kontrolle die Larven geschlüpft sind.

Bei diesem Versuch wurde die quantitative Schlupfhemmung beispielsweise mit den Verbindungen 128, 131, 132, 136 und 150 mit ≦ 0,5 Gew.% Wirkstoffaufbereitung erzielt.

Beispiel C

Prodenia litura (ägypt. Baumwollwurm); Zucht

Die Zucht erfolgt in 100 ml Plastikbechern auf ca. 50 ml des Standard-Nährbodens, dem im flüssigen Zustand der Wirkstoff sorgfältig untergemischt wurde. Je Konzentration wird 1 Becher mit 5 Raupen des vierten Larvenstadiums angesetzt. Die Versuchstemperatur beträgt 25 bis 26 °C. Die Beobachtung erstreckt sich bis zum Schlüpfen der Falter. Die Probe gilt als wirksam, wenn Riesenlarven produziert werden.

Bei diesem Versuch wurde eine quantitative Wirkung mit ≦ 0,5 ppm der Verbindungen 132 und 134 erzielt. Die Vergleichsverbindungen A, B und C zeigten bei 5,0 ppm keine Wirkung.

Beispiel D

Dysdercus intermedius (Baumwollwanze);, ovizide Wirkung

Stecketiketten werden am oberen Rand mit Klebestreifenstücken (ca. 0,8 cm) beklebt. 24 Stunden vor Versuchsbeginn werden die Eier durch Eintauchen in das "Sammelgefäß" an dem Klebestreifenrand befestigt. Die Eier werden für ca. 5 Sekunden in die wäßrige Wirkstoffaufbereitung getaucht. Dann läßt man auf Filterpapier abtropfen, ohne daß die Eier dabei auf das Papier gelegt werden. Die zugeschnittenen Etiketten werden in Plastikpaletten gestellt, mit den Klebestreifen nach oben. Eine halbe mit Wasser durchfeuchtete Watterolle wird dann in den Becher gelegt, um Austrocknung zu vermeiden, und die Palette wird mit einer Glasplatte abgedeckt. Nach ca. 8 Tagen wird bonitiert, wenn die Larven in der Kontrolle geschlüpft sind.

Bei diesem Test wurde eine sehr gute Schlupfhemmung mit 0,005 Gew.% der Verbindungen 128, 131, 132, 133 und 139 erzielt. Die Vergleichsverbindungen A, B und C zeigten bei 0,1 Gew.% keine Wirkung.

Beispiel E

Zuchtversuch mit Aedes aegypti (Gelbfiebermücken)

Bei 25 °C werden in einem 250 ml Plastikbecher 200 ml Leitungswasser mit der Wirkstoffaufbereitung versetzt und anschließend mit 20 bis 30 Moskitolarven im dritten bis vierten Larvenstadium besetzt. Während der Versuchsdauer wird einmal mit einem gepulverten, käuflichen Zierfischfutter (Tetramin®) gefüttert. Beobachtet werden Verpuppung und Schlupf der Imagines. Dies erfolgt nach ca. 10 bis 12 Tagen.

Bei diesem Versuch lag die mortale Dosis der Verbindung 9 und die der Vergleichsverbindungen A und C bei 5 ppm.

## Patentansprüche

**1.** N-substituierte Azole der allgemeinen Formel I

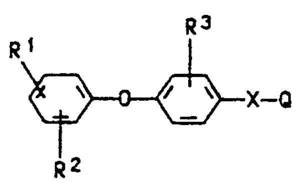

(I) ,

in der die Substituenten folgende Bedeutung haben:

$R^1$, $R^2$, $R^3$ Wasserstoff, Halogen, $C_1$-$C_8$-Alkyl, $C_1$-$C_8$-Alkoxy, $C_1$-$C_4$-Halogenalkyl $C_1$-$C_4$-Halogenalkoxy, $C_3$-$C_{10}$-Cycloalkyl, Nitro oder cyano,

Q ein gegebenenfalls substituierter Azolrest der Formeln IIa-IId

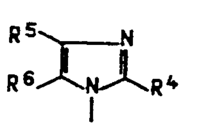

(IIa)

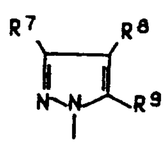

(IIb)

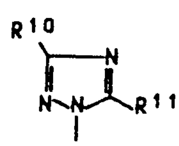

(IIc)

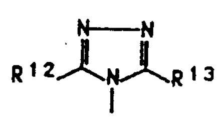

(IId)

in denen

$R^4$ bis $R^{13}$ Wasserstoff, Halogen, $C_1$-$C_8$-Alkyl, $C_1$-$C_4$-Halogenalkyl $C_1$-$C_4$-Alkoxy, $C_3$-$C_{10}$-Cycloalkyl, Phenyl, 1-Naphthyl oder 2-Naphthyl, wobei diese aromatischen Reste gegebenenfalls ein- bis dreifach durch Halogen, $C_1$-$C_8$-Alkyl, $C_1$-$C_8$-Alkoxy, $C_1$-$C_4$-Halogenalkyl oder $C_1$-$C_4$-Halogenalkoxy substituiert sind, bedeuten,

X im Falle des Azolrestes IIa $-CH_2-$ oder $-O(CH_2)_n-$, wobei n für 1, 2 oder 3 steht, im Falle der Azolreste IIb-IId $-OCH_2-$.

**2.** Verfahren zur Herstellung der N-substituierten Azole der allgemeinen Formel I gemäß Anspruch 1, dadurch gekennzeichnet, daß man in an sich bekannter Weise

a) für den Fall X = $-CH_2-$ eine p-Phenoxybenzylverbindung II

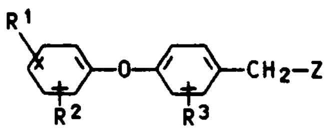

(III),

in der Z für eine Abgangsgruppe steht, mit dem Anion eines Imidazols IV

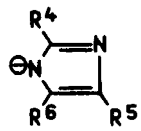

(IV)

oder

b) für den Fall X = $-O(CH_2)_2-$ und $-O(CH_2)_3$ eine p-Phenoxyphenoxyverbindung V

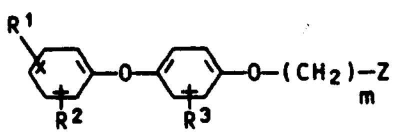

(V),

in der m für die Zahlen 2 oder 3 steht, mit dem Anion eines Imidazoles IV, oder

c1) für den Fall X = $-OCH_2-$ p-Phenoxyphenole VI

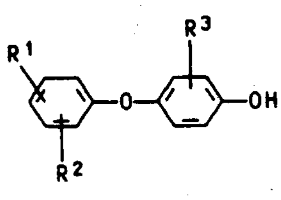

(VI)

mit einem N-Methylazol der Formel VII

$X-CH_2-Q$ (VII),

in der X eine Abgangsgruppe bedeutet und Q die in Anspruch 1 angegebenen Bedeutungen hat, in Gegenwart einer Base oder

c2) ein Phenolatanion von VIII

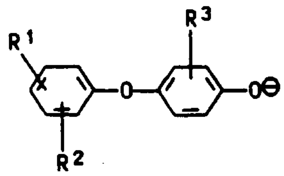

mit einem N-Methylazol VII umsetzt.

**3.** Schädlingsbekämpfungsmittel, enthaltend ein N-substituiertes Azol gemäß Anspruch 1 neben hierfür üblichen Trägerstoffen.

**4.** Schädlingsbekämpfungsmittel nach Anspruch 3, enthaltend 0,1 bis 95 Gew.% eines N-substituierten Azols der Formel I.

**5.** Verfahren zur Bekämpfung von Pflanzen Schädlingen, dadurch gekennzeichnet, daß man die Schädlinge und/oder die von Schädlingen freizuhaltenden Flächen und/oder Räume mit einer für Schädlinge wirksamen Menge eines N-substituierten Azols der Formel I gemäß Anspruch 1 behandelt.

**Claims**

**1.** An N-substituted azole of the general formula I

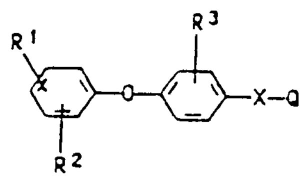

(I)

where $R^1$, $R^2$ and $R^3$ are each hydrogen, halogen, $C_1$-$C_8$-alkyl, $C_1$-$C_8$-alkoxy, $C_1$-$C_4$-haloalkyl, $C_1$-$C_4$-haloalkoxy, $C_3$-$C_{10}$-cycloalkyl, nitro or cyano,
Q is an unsubstituted or substituted azole radical of the formulae IIa-IId

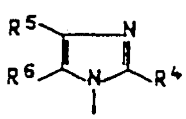

(IIa)

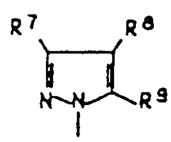

(IIb)

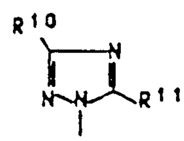

(IIc)

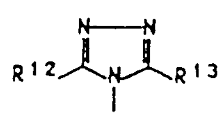

(IId)

where
$R^4$ to $R^{13}$ are each hydrogen, halogen, $C_1$-$C_8$-alkyl, $C_1$-$C_4$-haloalkyl, $C_1$-$C_4$-alkoxy, $C_3$-$C_{10}$-cycloalkyl, phenyl, 1-naphthyl or 2-naphthyl, said aromatic radicals being unsubstituted or mono-, di-or trisubstituted by halogen, $C_1$-$C_8$-alkyl, $C_1$-$C_8$-alkoxy, $C_1$-$C_4$-haloalkyl or $C_1$-$C_4$-haloalkoxy, and
X is, in the case of the azole radical IIa, $-CH_2-$ or $-O(CH_2)n-$, n being 1, 2 or 3, and, in the case of the azole radicals IIb-IId, $-OCH_2-$.

**2.** A process for the preparation of N-substituted azoles of the general formula I as claimed in claim 1, wherein

a) when X is $-CH_2-$ a p-phenoxybenzyl compound III

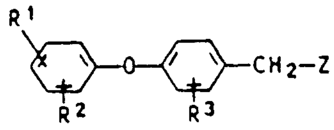

(III),

where Z is a leaving group, is reacted in conventional manner with the anion of an imidazole IV

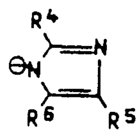

(IV)

or
b) when X is $-O(CH_2)_2-$ or $-O(CH_2)_3$ a p-phenoxyphenoxy compound V

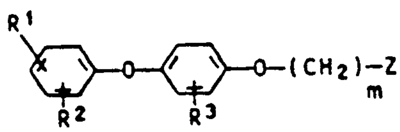

(V),

where m is one of the integers 2 and 3, is reacted in conventional manner with the anion of an imidazole IV, or
c1) when X is $-OCH_2-$ a p-phenoxyphenol VI

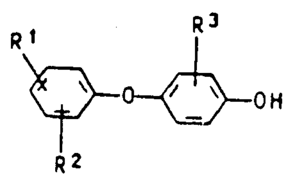

(VI)

is reacted in conventional manner with an N-methylazole of the formula VII

$X-CH_2-Q$ (VII),

where X is a leaving group and Q has the meanings given in claim 1, in the preonce of a base, or
c2) a phenolate anion of VIII

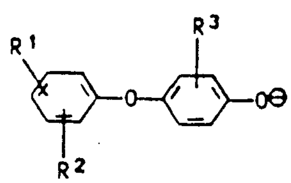

is reacted in conventional manner with an N-meth ylazole VII.

**3.** A pesticidal agent containing an N-substituted azole as claimed in claim 1, together with conventional carriers.

4. A pesticidal agent as claimed in claim 3, containing from 0.1 to 95 % by weight of an N-substituted azole of the formula I.

5. A method for controlling plant pests, wherein the pests and/or the areas and/or spaces to be kept free from pests are treated with a pesticidally effective amount of an N-substituted azole of the formula I as claimed in claim 1.

**Revendications**

1. Azoles substitués sur M, de la formule générale I

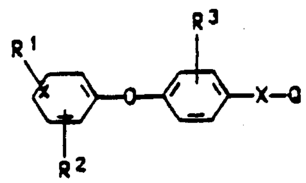

(I) ,

dans laquelle les substituants ont les significations suivantes :
$R^1$, $R^2$, $R^3$représentent hydrogène, halogène, alkyle en C1-C8, alcoxy en C1-C8, halogénoalkyle en C1-C4, halogénoalcoxy en C1-C4, cycloalkyle en C3-C10, nitro ou cyano,
Q est un reste azole éventuellement substitué dans des formules IIa-IId

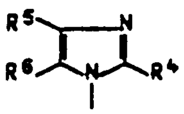

(IIa)

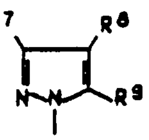

(IIb)

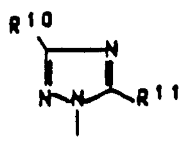

(IIc)

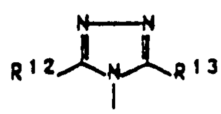

(IId)

dans lesquelles
$R^4$ à $R^{13}$ représentent hydrogène, halogène, alkyle en C1-C8, halogènealkyle en C1-C4, alcoxy en C1-C4, cycloalkyle en C3-C10, phényle, 1-naphtyle ou 2-naphtyle, ces restes aromatiques étant éventuellement substitués une ou trois foix par halogène, alkyle en C1-C8, alcoxy en C1-C8, halogénoalkyle en C1-C4 ou halogénoalcoxy en C1-C4,
X représente, dans le cas du reste azole IIa, $-CH_2$ ou $-O(CH_2)-$, n étant mis pour 1, 2 ou 3, et, dans le cas du reste azole IIb-IId, $-OCH_2-$.

2. Procédé de préparation des azoles substitués sur N de la formule générale I selon la revendication 1, caractérisé par le fait que l'on fait réagir, de manière connue en soi,
a) dans le cas où X = $-CH_2-$, un composé p-phénoxybenzylique III

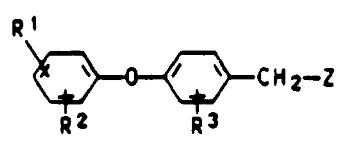

(III),

où Z est mis pour un groupe éliminable, avec l'anion d'un imidazole IV

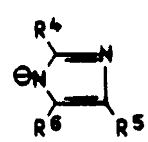

(IV)

ou
b) dans le cas où X = $-O(CH_2)_2-$ et $-O(CH_2)_3$, un composé p-phénoxyphénoxy V

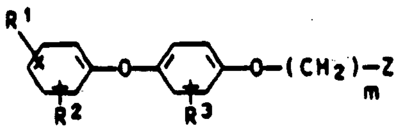

(V),

où m est pris pour les nombres 2 ou 3, avec l'anion d'un imidazole IV, ou
c1) dans le cas où X = $-OCH_2-$, des p-phénoxyphénoles VI

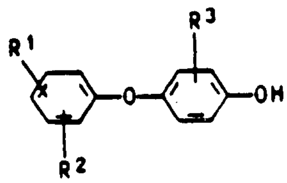

(VI)

avec un N-méthylazole de la formule VII

$X-CH_2-Q$ (VII)

où X représente un groupe éliminable et Q a des significations données dans la revendication 1, en présence d'une base ou
c2) un anion phénolate de VIII

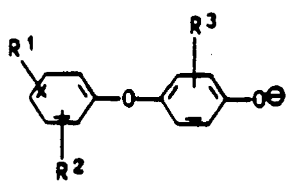

avec un N-méthylazole VII

**3.** Agent antiparasitaire, contenant un azole substitué sur N selon la revendication 1, outre des matières supports usuelles pour cela.

**4.** Agent antiparasitaire selon la revendication 3, contenant 0,1 à 95 % en poids d'un azole substitué sur N de la formule I.

**5.** Procédé de lutte contre les parasites des plantes caractérisé par le fait que l'on traite les parasites et/ou les surfaces et/ou espaces à maintenir exempts de parasites avec une quantité efficace contre les parasites d'un azole substitué sur N de la formule I selon la revendication 1.